Europäisches Patentamt

**European Patent Office**

**Office européen des brevets**

(11) Veröffentlichungsnummer: **0 238 918**
A1

# EUROPÄISCHE PATENTANMELDUNG

(21) Anmeldenummer: **87103261.1**

(22) Anmeldetag: **06.03.87**

(51) Int. Cl.⁴: **C 12 N 1/38,** C 12 N 1/36,
A 61 K 7/22, A 61 K 7/32,
A 61 K 7/48

(30) Priorität: **13.03.86 DE 3608423**

(43) Veröffentlichungstag der Anmeldung: **30.09.87**
**Patentblatt 87/40**

(84) Benannte Vertragsstaaten: **AT BE CH DE ES FR GB GR IT LI LU NL SE**

(71) Anmelder: **Probios Biotechnologie GmbH, Auf der Schanze 1, D-8490 Cham (DE)**

(72) Erfinder: **Becker, Klaus, Gojensbergsweg 89,, D-2050 Hamburg 80 (DE)**

(74) Vertreter: **Kuhnen, Wacker & Partner, Schneggstrasse 3-5 Postfach 1729, D-8050 Freising (DE)**

(54) Verfahren zur Steuerung stabiler mikrobieller Mischbiozönosen.

㉗ Durch die Zugabe von Supplinen und/oder Hemmstoffen, ggf. in Kombination mit bestimmten Nährstoffen kann die Zusammensetzung stabiler mikrobieller Mischbiozönosen, in denen Lactobacteriaceae (Milchsäuregärer) bestimmend sind, gesteuert werden. Hierdurch läßt sich die Bildung unerwünschter Stoffwechselprodukte der Bakterien, wie z.B. geruchsbildender Stoffe bei schweißabbauenden Bakterien oder die Kariesbildung begünstigende Laevanbildung durch Milchsäuregärer im Mund unterdrücken.

EP 0 238 918 A1

ACTORUM AG

0238918

KUHNEN · WACKER & PARTNER

PATENT- UND RECHTSANWALTSBÜRO

PATENTANWÄLTE · EUROPEAN PATENT ATTORNEYS
RAINER A. KUHNEN – Dipl.-Ing.
PAUL-ALEXANDER WACKER – Dipl.-Ing., Dipl.-Wirtsch.-Ing.
PETER FÜRNISS – Dr. Dipl.-Chem.

RECHTSANWALT
GÜNTER FRHR. v. GRAVENREUTH – Dipl.-Ing. (FH)
Zulassung: Bay. Oberstes Landesgericht,
OLG München, LG München I und II

Telefon: 0 81 61-62 09-1 · Telex: 526 547 pawa d
Telefax: 0 81 61-62 09-6 · Datex-P: 45-8 161-30 057
Teletex: 8 161 800=pawaMUC

D-8050 FREISING 1,    SCHNEGGSTRASSE 3-5

Probios Biotechnologie GmbH
8490 Cham

55PR07054-01
28.01.1987

# Verfahren zur Steuerung stabiler mikrobieller Mischbiozönosen

Die Erfindung betrifft ein Verfahren zur Steuerung der Zusammensetzung stabiler mikrobieller Mischbiozönosen, in denen Bakterien der Familie Lactobacteriaceae (Milchsäuregärer) bestimmend sind.

Beinahe alle mikrobiellen Stoffumwandlungen in der Natur erfolgen durch mikrobielle Mischbiozönosen (eine Mischbiozönose ist die Lebensgemeinschaft verschiedener Mikroorganismen). Auch viele biotechnische Stoffumwandlungen, z.B. die biologische Abwasserreinigung oder der Sauerteig beruhen auf den Stoffwechsel von mikrobiellen Mischbiozönosen. Es ist bekannt, daß sich die Mischbiozönosen in einem sehr langen Zeitraum - in Vergleich zur Generationszeit der einzelnen Arten - zu sehr stabilen (reifen, protektiven) Biozönosen entwickeln, bei denen die Orgnaisationshöhe, die z.B. als Verhältnis von Biomasse zu Produktivität ausgedrückt werden kann, groß ist. Dies gilt insbesondere für solche Biozönosen, deren Umweltbedingungen relativ konstant bleiben, wie dies bei homoisothermen Tieren (Warmblütlern) z.B. im Darm der Fall ist.

Stabile Biozönosen weisen im allgemeinen eine Vielzahl von Regelungsmechanismen auf, durch die Änderungen der äußeren Bedingungen abgefangen werden. Artenspektrum und Individuenzahl einer Biozönose pendeln in ihrem Klimaxstadium um einen Optimumpunkt. Erhöht sich beispielsweise das Nahrungsangebot, so vermehren sich vorübergehend fast nur die ubiquitären allochthonen Mikroorganismen, während die hochspezialisierten autochthonen fast konstant bleiben.

Diese autochthonen Mikroorganismen haben sich häufig so spezialisiert, daß sie zum Leben Suppline benötigen. Suppline sind Stoffe, die zum Grundbestand der Zelle gehören und von einzelnen Organismen nicht aus den einfachen Bausteinen synthetisiert werden können. Es handelt sich dabei um Aminosäuren, Purine, Pyrimidine, organische Säuren, Kohlenhydrate sowie Vitamine. Kohlenhydrate können sowohl Nährstoff als auch Supplin sein. Insbesondere selten vorkommende Kohlenhydrate können häufig als Supplin betrachtet werden. Von ihrer Funktion und Konzentration her unterscheiden sich Suppline deutlich von Nährstoffen. Sie entsprechen den Vitaminen bei der tierischen und menschlichen Ernährung. In mikrobiellen Mischbiozönosen dienen häufig End- oder Zwischenprodukte des Stoffwechsels einer Art der anderen als Suppline, wodurch sich ein Regelmechanismus ergibt (vgl. Schlegel, Allgemeine Mikrobiologie, 5. Auflage 1981, Stuttgart, Verlag Thieme, S. 169). Ein weiterer Regelmechanismus besteht darin, daß die einzelnen Mitglieder einer mikrobiellen Biozönose unterschiedlich empfindlich auf Hemmstoffe reagieren, die entweder von anderen Mitgliedern der mikrobiellen Biozönose oder aber auch von einem höheren Symbiosepartner, z. B. einer Wirtspflanze, stammen.

In Biozönosen werden die einzelnen Funktionen durch bestimmte Mitglieder dieser Biozönose ausgeübt, denen damit eine bestimmte ökologische Nische zukommt. In der Regel kann die gleiche ökologische Nische von mehreren Arten eingenommen werden. Es wurde nun gefunden, daß in mikrobiellen Mischbiozönosen, in denen Bakterien der Familie Lactobacteriaceae (Milchsäuregärer) bestimmend sind, deren Funktion für den Menschen von Interesse ist, einzelne Bakterienarten mit positiven Eigenschaften gezielt gefördert und/oder einzelne Mitglieder mit bestimmten negativen Eigenschaften gezielt unterdrückt werden können, wenn man die Tatsache nützt, daß viele autochthone Milchsäurebilder stabiler mikrobieller Mischbiozönosen eine unterschiedliche Bedürftigkeit gegenüber Supplinen und/oder Empfindlichkeit gegenüber biotischen Hemmstoffen aufweisen.

Häufig können Suppline einer Art in bestimmten Konzentrationen auf andere Arten hemmend wirken. Das gleiche Supplin kann auf die gleiche Art sowohl hemmend als auch fördernd wirken.

Der Erfindung liegt daher die Aufgabe zugrunde, in stabilen mikrobiellen Mischbiozönosen, in denen Bakterien der Familie Lactobacteriaceae bestimmend sind, eine oder mehrere ökologischen Nischen, die mit einer oder mehreren unerwünschten Bakterienarten besetzt sind, mit einer oder mehreren erwünschten Bakterienart(en) zu besetzen, deren ökologische Potenz ganz oder weitgehend der der unerwünschten Bakterienart(en) entspricht.

Die genannte Aufgabe wird bei einem Verfahren der eingangs genannten Art dadurch gelöst, daß man in einer oder mehreren ökologischen Nischen dieser Biozönose eine oder mehrere unerwünschte Bakterienart(en) durch eine oder mehrere erwünschte Bakterienart(en) ersetzt, indem man Suppline für die erwünschte(n) Art(en) und/oder Hemmstoffe für die unerwünschte(n) Art(en), ggf. in Kombination mit bestimmten Nährstoffen zugibt.

Die Stabilität der Biozönose verändert sich durch Suppline und/oder Hemmstoffe allein nicht oder nur unwesentlich, was ein wichtiger Vorteil des erfindungsgemäßen Verfahrens gegenüber dem Zusatz von Probiotika, künstlich gezüchteten, erwünschten Mikroorganismen (engl.: probiotics), ist. Durch Zugabe bestimmter geeigneter Nährstoffe wird eine Vermehrung der autochthonen Mikroorganismen ermöglicht. In diesem Vermehrungshub wird mit Supplinen und/oder Hemmstoffen eingegriffen. Dadurch wird es möglich, ökologische Nischen durch erwünschte Organismen zu besetzen. Wenn die Nährstoffe bereits vorgegeben sind, z. B. in bestimmten menschlichen Lebensmitteln, ist eine gesonderte Zugabe von Nährstoffen für Mikroorganismen überflüssig. Die ggf. vorteilhafte oder notwendige Zugabe von Nährstoffen kann gleichzeitig mit der Zugabe von Supplinen und/oder Hemmstoffen oder zeitlich unabhängig davon erfolgen.

Nach dem erfindungsgemäßen Verfahren können bestimmte erwünschte Spezies der Familie Lactobacteriaceae gefördert und andere gleichzeitig unterdrückt werden. Denn alle Lactobacteriaceae benötigen zum Wachstum Suppline.

In der Kombination von Supplinen mit einer Mischung von Kohlenhydraten und/oder einer organischen Stickstoffquelle lassen sich viele Milchsäuregärungen steuern. Hierzu gehören beispielsweise die Gärfutterbereitung aus grünen Pflanzenteilen (Erzeugung von Silage) aus Zuckerrübenblättern, Mais, Kartoffeln, Gras, Luzerne, die Herstellung wichtiger menschlicher Lebensmittel, wie Sauerteig, Sauerkraut, Sauerrahmbutter, Rohwurst (Salami, Cervelat), Quark, Harzer und Mainzer Käse, Hartkäse, Buttermilch usw. Ferner kann durch eine Steuerung der Lactobacteriaceae in die Mikroflora der Nasenhöhle von Tier und Mensch eingegriffen werden.

Durch Unterdrückung bestimmter Milchsäuregärer kann man in der Mundhöhle die Kariesbildung verhindern. Erfindungsgemäß macht man Suppline für erwünschte Lactobacteriaceae und/oder Hemmstoffe für unerwünschte Lactobacteriaceae mit verschiedenen Mitteln in der Mundhöhle verfügbar. Hierbei handelt es sich um die folgenden Mittel:

Zahnpasta und Zahnpulver
Mundwässer, Kaugummi
Mundpillen, Konfekt und Süßwaren, zahnumhüllende Konzentrate
orale Tabletten mit verzögerter Freigabe des Wirkstoffs.

Bei der menschlichen Haut ist es möglich, durch geeignete Supplinkombination die schweißabbauenden Bakterien so zu steuern, daß zwar das "Säureschutzschild" erhalten bleibt, jedoch möglichst wenig geruchsbildende Stoffe entstehen.

Nährstoffe für Bakterien der Familie Lactobacteriaceae sind an sich bekannt (vgl. Schlegel, Allgemeine Mikrobiologie usw. (s. oben), S. 176). Hierzu gehören insbesondere Kohlenhydrate.

In den obengenannten Anwendungsfällen auftretende unerwünschte Milchsäuregärer (Lactobacteriaceae), und für erwünschte Milchsäuregärer notwendige Suppline sind in der folgenden Tabelle 1 zusammengefaßt:

Tabelle 1

| Reaktion | unerwünschte Milchsäuregärer | erwünschte Milchsäuregärer derselben ökologischen Nische | Suppline hierfür |
|---|---|---|---|
| Gärfutterbereitung | | Lactobacilli | Nikotinsäure |
| Kariesbildung | Streptococcus mutans S. salivarius | Lactobacteriaceae spec. | Glutamin Alanin Arginin Asparagin Glycin Isoleucin Leucin Lysin Phenylalanin Prolin Serin Threonin Valin Nicotinsäure Cholin Riboflavin Panthothensäure Inosit |

Beispiel 1: <u>Gärfutterbereitung.</u>

Silage von Silomais wurde im Laborversuch angesetzt, wobei (wie in der Praxis) kein voller Luftabschluß gegeben war. Das erwünschte Sinken des pH-Werts (als Indikator für die Milchsäurebildung) erfolgte ohne Zusätze wesentlich langsamer als mit Zusatz geeigneter Kombinationen aus einem Supplin und bestimmten Nährstoffen. Bei Zugabe einer Mischung von Nikotinsäure, Xylose und Mannit (1:100:75) in einer Menge von 0,7 g/kg Trokkensubstanz am ersten Tag des Versuchs wurde bereits nach 15 Tagen ein pH-Wert von 4,1 erreicht gegenüber einem pH-Wert von 5,9 nach 15 Tagen bei der Probe ohne Zusätze.

Beispiel 2: <u>Verhinderung der Kariesbildung (Mundwasser).</u>

Eine wichtige Voraussetzung für die Kariesbildung sind Milchsäuregärer (z. B. Streptococcus mutans und S. salivarius), die gleichzeitig Saccharose in Laevane umsetzen. Durch Spülen der menschlichen Mundhöhle mit einer wässrigen Aufschwemmung von hochdisperser Kieselsäure (als Aufwuchssubstrat) mit einem geeigneten Präparat, das einen Hemmstoff für Hexosyltransferase bildende Arten von Mikroorganismen enthält, läßt sich die Hexosyltransferaseausscheidung im Ausstrich des Zahnbelags vermindern. Das verwendete Präparat enthielt neben den üblichen Bestandteilen (alkoholischen Lösungen von etherischen Ölen, Netzmitteln, Adstringentien und Antiseptika) die folgende Wirkstoffkombinationen pro Liter wässrige Zubereitung:

8

| Glutamin | 24 g |
| EDTA | 60 g |
| Alanin | 1 000 mg |
| Arginin | 625 mg |
| Asparagin | 1 400 mg |
| Glycin | 720 mg |
| Isoleucin | 720 mg |
| Leucin | 900 mg |
| Lysin | 1 000 mg |
| Phenylalanin | 500 mg |
| Prolin | 500 mg |
| Serin | 720 mg |
| Threonin | 720 mg |
| Valin | 720 mg |
| Nicotinsäure | 24 mg |
| Cholin | 46 mg |
| Riboflavin | 10 mg |
| Panthothensäure | 10 mg |
| Inosit | 100 mg. |

Mit 100 ml dieser Lösung spülte der Proband morgens vor dem Zähneputzen den Mund. Aus dem Ausgespülten wurden die Hexosyltransferasebildner bestimmt.

a) Bestimmung der Hexosyltransferasebildner

Pro Proband wurden 15 kleine Petrischalen mit 1,5 % Agar (2 % Succrose, 2 % Hefeextrakt) zu je 5 ml gefüllt. Von dem Ausgespülten werden in aqua deion. steril. Verdünnungen in folgenden Konzentrationen hergestellt:

1:1000, 1:10 000, 1:100 000, 1:1 000 000, 1:10 000 000.

Nach 24 Stunden bei 37°C erfolgt die Auszählung der Kolonien (Hexosyltransferasebildner). Alle Verdünnungen werden in drei Parallel-Versuchen bestimmt. Aus den Petrischalen wird mit dem Lederbergstempel auf Mannit-Agar (2 %) überimpft und erneut 24 Stunden bei 37°C bebrütet (Streptococcus mutans). Die Ergebnisse sind in der folgenden Tabelle 2 enthalten. Die Reduktion der Hexosyltransferasebildner wird im Vergleich zum nichttherapierten Probanden bestimmt.

Ergebnis:

Innerhalb von 4 Tagen sinkt die Anzahl der Hexosyltransferasebildner durchschnittlich auf 1,1 %, wobei der Wert für die Bakterienart S.mutans durchschnittlich auf $1,5 \times 10^{-3}$ sinkt.

Tabelle 2

| Proband Nr. | | 1 | 2 | 3 | 4 | 5 | 6 | 7 | 8 |
|---|---|---|---|---|---|---|---|---|---|
| zu Beginn des Versuches | HTB | $1,2 \cdot 10^7$ | $8,6 \cdot 10^6$ | $7,8 \cdot 10^6$ | $2,1 \cdot 10^7$ | $4,5 \cdot 10^6$ | $1,2 \cdot 10^7$ | $2,6 \cdot 10^7$ | $8,2 \cdot 10^6$ |
| | Mann.pos. | $0,4 \cdot 10^7$ | $1,8 \cdot 10^5$ | $8,0 \cdot 10^5$ | $1,4 \cdot 10^6$ | $5,2 \cdot 10^5$ | $6,0 \cdot 10^6$ | $6,2 \cdot 10^6$ | $1,5 \cdot 10^6$ |
| nach 4 Tagen | HTB | $7,1 \cdot 10^4$ | $1,3 \cdot 10^5$ | $2,7 \cdot 10^4$ | $6,2 \cdot 10^5$ | $5,2 \cdot 10^4$ | $1,2 \cdot 10^4$ | $4,6 \cdot 10^5$ | $1,7 \cdot 10^4$ |
| | Mann.pos. | $1,2 \cdot 10^2$ | $1,0 \cdot 10^3$ | $1,6 \cdot 10^3$ | $2,3 \cdot 10^3$ | $4,2 \cdot 10^2$ | $2,4 \cdot 10^3$ | $8,2 \cdot 10^2$ | $1,0 \cdot 10^3$ |

HTB = Hexosyltransferasebildner

Mann.positiv $\approx$ S.mutans

11

Beispiel 3: <u>Verhinderung der Kariesbildung (Zahnpasta)</u>

Eine Zahnpasta aus einer Grundmasse nach dem Stand der Technik aus:

33 % Calciumphosphat,
20 % Glycerin,
1,6 % Natriumlaurylsulfat,
Rest Wasser,

enthält pro Kilogramm Zahnpastagrundmasse bzw. pro Dosis (= 2 g Zahnpasta) die folgenden Substanzen:

|                   | pro kg    | pro Dosis   |
|-------------------|-----------|-------------|
| Glutamin          | 24 g      | 48 mg       |
| EDTA              | 60 g      | 120 mg      |
| Alanin            | 1 000 mg  | 2 mg        |
| Arginin           | 625 mg    | 1,2 mg      |
| Asparagin         | 1 400 mg  | 2,8 mg      |
| Glycin            | 720 mg    | 1,4 mg      |
| Isoleucin         | 720 mg    | 1,4 mg      |
| Leucin            | 900 mg    | 1,8 mg      |
| Lysin             | 1 000 mg  | 2 mg        |
| Phenylalanin      | 500 mg    | 1 mg        |
| Prolin            | 500 mg    | 1 mg        |
| Serin             | 720 mg    | 1,4 mg      |
| Threonin          | 720 mg    | 1,4 mg      |
| Valin             | 720 mg    | 1,4 mg      |
| Nicotinsäure      | 24 mg     | 48 µg       |
| Cholin            | 46 mg     | 92 µg       |
| Riboflavin        | 10 mg     | 20 µg       |
| Panthothensäure   | 10 mg     | 20 µg       |
| Inosit            | 100 mg    | 200 µg.     |

KUHNEN · WACKER & PARTNER
PATENT- UND RECHTSANWALTSBÜRO

PATENTANWÄLTE · EUROPEAN PATENT ATTORNEYS
RAINER A. KUHNEN — Dipl.-Ing.
PAUL-ALEXANDER WACKER — Dipl.-Ing., Dipl.-Wirtsch.-Ing.
PETER FÜRNISS — Dr. Dipl.-Chem.

RECHTSANWALT
GÜNTER FRHR. v. GRAVENREUTH — Dipl.-Ing. (FH)
Zulassung:  Bay. Oberstes Landesgericht,
        OLG München, LG München I und II

Telefon: 0 81 61-62 09-1 · Telex: 526 547 pawa d
Telefax: 0 81 61-62 09-6 · Datex-P: 45-8 161-30 057
Teletex: 8 161 800=pawaMUC

D-8050 FREISING 1,    SCHNEGGSTRASSE 3-5

Probios Biotechnologie GmbH

8490 Cham

Bundesrepublik Deutschland

55PR07054-02

22.01.1987

## Patentansprüche

1. Verfahren zur Steuerung der Zusammensetzung stabiler mikrobieller Mischbiozönosen, in denen Bakterien der Familie Lactobacteriaceae (Milchsäuregärer) bestimmend sind, dadurch gekennzeichnet, daß man in einer oder mehreren ökologischen Nischen dieser Biozönose eine oder mehrere unerwünschte Art(en) von Milchsäuregärern durch eine oder mehrere erwünschte Art(en) von Milchsäuregärern ersetzt, indem man Suppline für die erwünschte(n) Art(en) und/ oder Hemmstoffe für die unerwünschte(n) Art(en), ggf. in Kombination mit bestimmten Nährstoffen zugibt.

2. Verfahren zur Steuerung stabiler mikrobieller Mischbiozönosen nach Anspruch 1, dadurch gekennzeichnet, daß man in der Mundhöhle Hexosyltransferasen bildende Arten von Lactobacteriaceae durch solche Arten von Lactobacteriaceae ersetzt, die keine oder wenig Hexosyltransferasen bilden.

22.01.1987                                KW&P: 55PR07054-02

2

3.  Verfahren nach Anspruch 2, dadurch gekennzeichnet,
    daß man Hexosyltransferase bildende Arten der Gattung Streptococcus, insbesondere Str. mutans, durch
    Inkontaktbringen der Mundhöhle mit Hemmstoffen
    hierfür und/oder mit Supplinen für andere Mikroor-
    ganismen durch andere Arten der Familie Lactobacteriaceae ersetzt.

4.  Verfahren zur Steuerung stabiler mikrobieller
    Mischbiozönosen nach Anspruch 1, dadurch gekennzeichnet, daß man auf der Haut die schweißabbauenden
    Bakterien, die geruchsintensive End- oder Nebenprodukte bilden, durch solche ersetzt, die geruchsfreie
    oder geruchsarme End- oder Nebenprodukte bilden,
    ohne daß der pH-Wert der Hautoberfläche ungünstig
    verändert wird.

5.  Verfahren nach Anspruch 2 und/oder 3, dadurch gekennzeichnet, daß man die Mundhöhle mit einer Glutamin, EDTA, Aminosäuren und Vitamine enthaltenden
    Mischung in Kontakt bringt.

6.  Verfahren nach Anspruch 5, dadurch gekennzeichnet,
    daß man die Zähne mit einer Zahnpasta putzt, die auf
    1 kg Grundmasse neben üblichen Bestandteilen die
    folgenden Stoffe enthält:

         24 g   Glutamin
         60 g   EDTA
      1 000 mg  Alanin
        625 mg  Arginin
      1 400 mg  Asparagin
        720 mg  Glycin
        720 mg  Isoleucin
        900 mg  Leucin
      1 000 mg  Lysin

    500 mg Phenylalanin

    500 mg Prolin

    720 mg Serin

    720 mg Threonin

    720 mg Valin

     24 mg Nicotinsäure

     46 mg Cholin

     10 mg Riboflavin

     10 mg Panthothensäure

    100 mg Inosit.

7. Mittel zur Verhinderung der Kariesbildung, enthaltend Glutamin, EDTA, Aminosäuren und Vitamine.

8. Mittel zur Verhinderung der Kariesbildung nach Anspruch 7, dadurch gekennzeichnet, daß es als Aminosäuren und Vitamine die folgenden Verbindungen enthält:

Alanin

Arginin

Asparagin

Glycin

Isoleucin

Leucin

Lysin

Phenylalanin

Prolin

Serin

Threonin

Valin

Nicotinsäure

Cholin

Riboflavin

Panthothensäure

Inosit.

9. Zahnpasta, enthaltend auf 1 kg Grundmasse neben
   üblichen Bestandteilen:

    24 g   Glutamin

    60 g   EDTA

1 000 mg   Alanin

  625 mg   Arginin

1 400 mg   Asparagin

  720 mg   Glycin

  720 mg   Isoleucin

  900 mg   Leucin

1 000 mg   Lysin

  500 mg   Phenylalanin

  500 mg   Prolin

  720 mg   Serin

  720 mg   Threonin

  720 mg   Valin

   24 mg   Nicotinsäure

   46 mg   Cholin

   10 mg   Riboflavin

   10 mg   Panthothensäure

  100 mg   Inosit.

## Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-2 542 886 (E.C. WACH)<br>* Spalte 1, Zeile 46 - Spalte 2, Zeile 21; Spalte 4, Zeilen 7-12 * | 1-3 | C 12 N 1/38<br>C 12 N 1/36<br>A 61 K 7/22<br>A 61 K 7/32<br>A 61 K 7/48 |
| Y | --- | 1-9 | |
| X | US-A-4 356 190 (K.S. KRASKIN)<br>* Spalte 1, Zeile 68 - Spalte 2, Zeile 7; Spalte 3, Zeile 65 - Spalte 4, Zeile 15; Spalte 5, Zeilen 1-66; Spalte 6, Zeilen 26-68; Spalte 7, Zeilen 3-9; Ansprüche * | 1,4-6 | |
| Y | --- | 1-5 | RECHERCHIERTE SACHGEBIETE (Int. Cl.4) |
| A | PATENT ABSTRACTS OF JAPAN, Band 6, Nr. 85 (C-103)[963], 22. Mai 1982; & JP-A-57 18982 (DAIICHI SEIYAKU K.K.) 30.01.1982<br>* Zusammenfassung *<br>--- | 1-3 | A 61 K<br>C 12 N |
| A | CHEMICAL ABSTRACTS, Band 98, Nr. 19, Mai 1983, Seite 386, Zusammenfassung Nr. 159130n, Columbus, Ohio, US; & JP-A-58 16 679 (YAKULT HONSHA CO., LTD.) 31.01.1983<br>* Zusammenfassung *<br>--- -/- | 1-3 | |

Der vorliegende Recherchenbericht wurde für alle Patentansprüche erstellt.

| Recherchenort | Abschlußdatum der Recherche | Prüfer |
|---|---|---|
| DEN HAAG | 09-07-1987 | BERTE M.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

**Europäisches Patentamt**

# EUROPÄISCHER RECHERCHENBERICHT

EP 87 10 3261

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE-A-3 043 792 (R.E. GUNTHER) <br> * Ansprüche * <br> --- <br> ----- | 1-9 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 09-07-1987 | BERTE M.J. |